# EUROPEAN PATENT APPLICATION

(11) **EP 3 437 597 A1**
(43) Date of publication of application: **06.02.2019**
(21) Application number: 16896716.4
(22) Date of filing: 28.03.2016
(51) Int. Cl.: A61F 13/15, B65B 41/16, B65B 57/00

(54) **METHOD FOR MANUFACTURING AND DEVICE FOR MANUFACTURING PACKAGE FOR ABSORBENT ARTICLE**

(71) Applicant: Unicharm Corporation, Shikokuchuo-shi, Ehime 799-0111 (JP)
(72) Inventor: SAEKI, Tatsuya, Kanonji-shi Kagawa 769-1602 (JP)
(74) Representative: Staeger & Sperling Partnerschaftsgesellschaft mbB
(86) International application number: PCT/JP2016/059830
(87) International publication number: WO 2017/168496

(57) **Abstract**

A method for manufacturing a package (7p) formed by wrapping an absorbent article (1) with a packaging sheet (7), the method comprising: a first transport process of transporting a plurality of the absorbent articles (1) at a first pitch (P1), a second transport process of transporting a continuous sheet (7a) of packaging sheets (7) in a direction of transport toward a gathering position, the gathering position (Pj) being a position where the continuous sheet and the absorbent articles (1) are gathered, and a joining process of joining each of the absorbent articles (1) to the continuous sheet (7a) at the gathering position (Pj), the second transport process comprising a state-of-stretch adjusting process of acquiring information on a difference of the center-to-center interval between the mark elements (M1) from the first pitch (P1), and adjusting a state of stretch of the continuous sheet (7a) using a state-of-stretch adjusting device (41) based on the information on the difference of the center-to-center interval, and a transport speed value adjusting process of acquiring information on a distance of a position of one of the mark elements from a reference position, the reference position being based on the first pitch (P1) on the continuous sheet (7a) and adjusting, based on the information on the distance of the position, a transport speed value at a time when a delivering device (45) delivers the continuous sheet (7a) toward the gathering position (Pj).

## Description

### [Technical Field]

The present disclosure relates to a method and apparatus for manufacturing a package of an absorbent article such as a sanitary napkin.

### [Background Art]

Sanitary napkins serving as an example of absorbent articles are supplied to the market in the form of packages formed by individually wrapping the sanitary napkins with packaging sheets, respectively.

### [Citation List]

### [Patent Literature]

[PTL 1] Japanese Patent Application Laid-open Publication No. 2012-75590

### [Summary]

### [Technical Problem]

In general, patterns, such as an illustration, a product name, and/or a design, are printed on the packaging sheet. Such packages are formed, for example, as follows.

First, an appropriate transporting apparatus, such as a conveyer, is used to transport napkins while being aligned in a direction of transport at product pitches. On the other hand, a continuous sheet of the packaging sheet is also transported in the direction of transport using an appropriate transporting apparatus such as conveyor rollers, and patterns are printed on the sheet while being aligned in the direction of transport at print pitches smaller than the product pitches.

Thus, when napkins are disposed on the continuous sheet, the continuous sheet is stretched by a difference between the product pitch and the print pitch, so that the positions of the patterns on the continuous sheet are matched with the positions of the napkins.

Then, the napkins are disposed on the sheet stretched as such, and both lateral ends of the sheet are folded inward in a tri-folded state. Further, a pair of sealed portions is formed in the tri-folded sheet, and furthermore the sheet is cut at a position between a pair of sealed portions, to produce packages.

However, the aforementioned state of stretch can change over time due to various factors. When the state of stretch changes, it becomes difficult to dispose the napkins at the appropriate positions on the continuous sheet determined by the positional relationship with the above-described patterns. The positions at which such absorbent articles are disposed deviate from the aforementioned appropriate positions, which results in generally unattractive packages.

The present disclosure has achieved in light of the aforementioned conventional issues, and an object of the present disclosure is to make it possible to dispose an absorbent article in an appropriate position on a continuous sheet of a packaging sheet, thereby being able to manufacturing a package having preferable appearance.

### [Solution to Problem]

A main aspect of the disclosure for achieving an object described above is a method for manufacturing a package formed by wrapping an absorbent article with a packaging sheet, the method comprising: a first transport process of transporting a plurality of absorbent articles at a first pitch, a second transport process of transporting a continuous sheet of packaging sheets in a direction of transport toward a gathering position, the gathering position being a position where the continuous sheet and the absorbent articles are gathered, the continuous sheet having mark elements wherein the mark elements are aligned in the direction of transport at a center-to-center interval that is smaller than the first pitch when the continuous sheet is in an unstretched state, and a joining process of joining each of the absorbent articles to the continuous sheet at the gathering position, the second transport process comprising a state-of-stretch adjusting process of acquiring information on a difference of the center-to-center interval between the mark elements from the first pitch, and adjusting a state of stretch of the continuous sheet using a state-of-stretch adjusting device based on the information on the difference of the center-to-center interval, and a transport speed value adjusting process of acquiring information on a distance of a position of one of the mark elements from a reference position, the reference position being based on the first pitch on the continuous sheet and adjusting, based on the information on the distance of the position, a transport speed value at a time when a delivering device delivers the continuous sheet toward the gathering position.

Further, an apparatus for manufacturing a package formed by wrapping an absorbent article with a packaging sheet, the apparatus comprising: a first transporting apparatus for transporting a plurality of absorbent articles at a first pitch; a second transporting apparatus for transporting a continuous sheet of packaging sheets in a direction of transport toward a gathering position, the gathering position being a position where the continuous sheet and the absorbent articles are gathered, the continuous sheet having mark elements wherein the mark elements are aligned in the direction of transport at a center-to-center interval that is smaller than the first pitch when the continuous sheet is in an unstretched state; and a joining device for joining each of the absorbent articles to the continuous sheet at the gathering position, the second transporting apparatus comprising a state-of-stretch adjusting device for acquiring information on a difference of the center-to-center interval between the mark elements from the first pitch, and adjusting a state of stretch of the continuous sheet based on the information on the difference of the center-to-center interval; and a delivering device for acquiring information on a distance of a position of one of the mark elements from a reference position, the reference position being based on the first pitch on the continuous sheet, and adjusting, based on the information on the distance of the position, a transport speed value at a time when delivering the continuous sheet toward the gathering position.

Other features of the present disclosure will become apparent from the descriptions of the present specification with reference to the accompanying drawings.

### [Advantageous Effects]

According to the present disclosure, it is possible to dispose an absorbent article in an appropriate position on a continuous sheet of a packaging sheet, to produce a package having a preferable appearance.

### [Brief Description of Drawings]

FIG. 1A is a schematic plan view illustrating a sanitary napkin 1 as an example of an absorbent article. FIG. 1B is a cross sectional view taken along line B-B of FIG. 1A.
FIG. 2A is a schematic perspective view illustrating a napkin 1 placed on a packaging sheet 7. FIG. 2B is a schematic plan view illustrating a package 7p obtained by packaging a napkin 1 with a packaging sheet 7. FIG. 2C is a schematic plan view illustrating the package 7p when viewed from the side opposite to the side as in FIG. 2B.
FIG. 3 is a schematic plan view illustrating a process of forming a package 7p of a napkin 1.
FIG. 4 is a schematic side view illustrating a production line LM for a package 7p of a napkin 1.
FIG. 5 is a schematic diagram for explaining a direction changing process and a pitch changing process performed by a state-of-napkin changing device 21.
FIG. 6 is a schematic side view of an example of a state-of-napkin changing device 21 having a direction changing function and a pitch changing function.
FIG. 7 is a schematic plan view illustrating a continuous sheet 7a of a packaging sheet.
FIG. 8 is a schematic plan view illustrating a continuous sheet 7a of a packaging sheet.
FIG. 9 is a schematic side view illustrating a tape member attaching device 71.
FIG. 10 is a schematic side view illustrating a seal cutting device 91.
FIG. 11 is a schematic side view illustrating a production line LM of a first modification.
FIG. 12 is a schematic side view illustrating a production line IM of a second modification.

### [Description of Embodiments]

At least following matter will become clear from the descriptions of the present specification with reference to the accompanying drawings.

A method for manufacturing a package formed by wrapping an absorbent article with a packaging sheet, the method comprising: a first transport process of transporting a plurality of absorbent articles at a first pitch, a second transport process of transporting a continuous sheet of packaging sheets in a direction of transport toward a gathering position, the gathering position being a position where the continuous sheet and the absorbent articles are gathered, the continuous sheet having mark elements wherein the mark elements are aligned in the direction of transport at a center-to-center interval that is smaller than the first pitch when the continuous sheet is in an unstretched state, and a joining process of joining each of the absorbent articles to the continuous sheet at the gathering position, the second transport process comprising a state-of-stretch adjusting process of acquiring information on a difference of the center-to-center interval between the mark elements from the first pitch, and adjusting a state of stretch of the continuous sheet using a state-of-stretch adjusting device based on the information on the difference of the center-to-center interval, and a transport speed value adjusting process of acquiring information on a distance of a position of one of the mark elements from a reference position, the reference position being based on the first pitch on the continuous sheet and adjusting, based on the information on the distance of the position, a transport speed value at a time when a delivering device delivers the continuous sheet toward the gathering position.

According to such a method for manufacturing a package of an absorbent article, since a transport speed value of the continuous sheet is changed based on the information on the distance of the position described above, it is possible to adjust the positions of the mark elements such that the positions in the direction of transport of the mark elements are matched with the aforementioned reference positions. Further, since the state of stretch of the continuous sheet is changed based on the information on the difference of the center-to-center interval, it is possible to make adjustment such that the aforementioned distance of the center-to-center interval will be the first pitch that is a transport pitch of the absorbent articles. Accordingly, it becomes possible to dispose the absorbent article in the appropriate position on the continuous sheet of the packaging sheet, thereby being able to produce a package having a preferable appearance.

The absorbent article is joined to the continuous sheet. Thus, even in the case where the continuous sheet is subjected to an appropriate process thereafter, the position of the absorbent article is maintained in the appropriate position on the continuous sheet. This also contributes to manufacturing of packages having preferable appearances.

In such a method for manufacturing a package of an absorbent article, it is preferable to further include a tri-folding process of folding both lateral ends of the continuous sheet inward together with the absorbent articles using a folding device so as to form the continuous sheet in a tri-folded state, when the continuous sheet passes a position downstream in the direction of transport from the gathering position.

According to such a method for manufacturing a package of an absorbent article, it is possible to produce a package in a tri-folded form. Further, since the continuous sheet and the absorbent articles are joined together, even when they are tri-folded, relative positional relationship is maintained. As a result, it becomes possible to maintain the absorbent article in the appropriate position on the continuous sheet.

In such a method for manufacturing a package of an absorbent article, it is preferable to further include a third transport process of transporting the tri-folded continuous sheet in the direction of transport, wherein the tri-folded continuous sheet has a plurality of second mark elements aligned in the direction of transport corresponding to the absorbent articles, the third transport process comprises a second position distance information acquisition process of acquiring information on a distance of a position of one of the second mark elements from a reference position, the reference position being based on the first pitch on the tri-folded continuous sheet, and a fixing process of fixing the continuous sheet in the tri-folded state by attaching a tape member to the tri-folded continuous sheet using a tape-member attaching device, when the tri-folded continuous sheet passes a predetermined position in the direction of transport, the tape-member attaching device is used to deliver the tape member to the predetermined position by rotating a rotating body of the tape-member attaching device while holding the tape member on an outer circumferential surface of the rotating body, and timing in which the tape member is delivered to the predetermined position is shifted from timing indicated by a synchronization signal for processing the continuous sheet at the first pitch, based on the information on the distance.

According to such a method for manufacturing a package of an absorbent article, the information on the distance of the position of the second mark element from the reference position that is based on the first pitch on the continuous sheet is acquired, and based on the information, timing in which the tape member is delivered to the predetermined position is shifted from timing indicated by the aforementioned synchronization signal. Accordingly, it becomes possible to attach the tape member to an appropriate position on the continuous sheet in the tri-fold state.

In such a method for manufacturing a package of an absorbent article, it is preferable to further include a third transport process of transporting the tri-folded continuous sheet in the direction of transport, wherein the tri-folded continuous sheet has a plurality of second mark elements aligned in the direction of transport corresponding to the absorbent articles, the third transport process comprises a second position distance information acquisition process of acquiring information on a distance of a position of one of the second mark elements from a reference position, the reference position being based on the first pitch on the tri-folded continuous sheet, and a forming process of forming the package such that while a pair of sealed portions is formed in the tri-folded continuous sheet using a sealing cutting device, the continuous sheet is cut at a position between the pair of sealed portions using the sealing cutting device, and in the forming process, timing in which the sealed portions are formed and timing in which the continuous sheet is cut by the sealing cutting device are shifted from timing indicated by a synchronization signal for processing the continuous sheet at the first pitch, based on the information on the distance.

According to such a method for manufacturing a package of an absorbent article, the information on the distance of the position of the second mark element from the reference position that is based on the aforementioned first pitch on the continuous sheet is obtained, and based on the information, timing in which the sealed portions are formed and timing in which the continuous sheet is cut are shifted from timing indicated by the aforementioned synchronization signal. Accordingly, it becomes possible to form the sealed portion at an appropriate position on the continuous sheet in the tri-fold, and also cut the continuous sheet at an appropriate position.

In such a method for manufacturing a package of an absorbent article, it is preferable that in the tri-folding process, the folding device is used to fold both the lateral ends inward along folding lines defined in two positions in a width direction in the continuous sheet such that the continuous sheet is formed in the tri-folded state, the second mark elements are provided in a position between the folding lines in the continuous sheet, and information on the distance of the position of one of the second mark elements is acquired in the second position distance information acquisition process by imaging the one of the second mark elements in the tri-folded continuous sheet using an imaging device.

According to such a method for manufacturing a package of an absorbent article, the second mark elements are provided in the position between the above-described folding lines. This position is less affected by folding when tri-folding. Accordingly, the imaging device can stably image at least one of the second mark elements stably.

In such a method for manufacturing a package of an absorbent article, it is preferable that the absorbent article and the continuous sheet are joined to each other with an adhesive in the joining process, information on the distance of the position of one of the mark elements and information on the difference of the center-to-center interval are acquired by imaging the mark elements on the continuous sheet, in a predetermined imaging position in the direction of transport using an imaging device, and the adhesive is applied onto the continuous sheet in a position between the imaging position and the gathering position in the direction of transport using an adhesive applying device.

According to such a method for manufacturing a package of an absorbent article, an adhesive is applied to the continuous sheet at a position close to the gathering position. Accordingly, it becomes possible to reduce change in the direction of transport of the position of the applied adhesive, thereby being able to reliably join the absorbent article to the continuous sheet with the adhesive.

In such a method for manufacturing a package of an absorbent article, it is preferable to further include a connecting process of, when the continuous sheet fed from a material coil is defined as a preceding continuous sheet, connecting a leading end in feeding of a succeeding continuous sheet fed from another material coil to the preceding continuous sheet, wherein
in the connecting process, the succeeding continuous sheet is connected to the preceding continuous sheet while performing registration such that a distance in the direction of transport between a predetermined position of one of the mark elements in the preceding continuous sheet and a position corresponding to the predetermined position of one of the mark elements in the succeeding continuous sheet falls within ±5% of the first pitch.

According to such a method for manufacturing a package of an absorbent article, the preceding continuous sheet and the succeeding continuous sheet are connected with such a positional accuracy that the distance in the direction of transport between one of the mark elements of the preceding continuous sheet and one of the mark elements of succeeding continuous sheet falls within ±5% of the aforementioned second pitch. Accordingly, the continuity brought by the connection between the preceding continuous sheet and the succeeding continuous sheet also substantially ensures the state of alignment in the mark elements. This makes it possible that the state-of-stretch adjusting device stably adjusts the state of stretch even on the border between the preceding continuous sheet and the succeeding continuous sheet, and also the delivering device stably adjust a transport speed value.

In such a method for manufacturing a package of an absorbent article, it is preferable that in the first transport process, a direction changing device is used to receive the absorbent articles transported from an upstream side in the direction of transport, with a longitudinal direction of the absorbent articles being along the direction of transport, the direction changing device is used to change a direction of the received absorbent articles such that a lateral direction intersecting the longitudinal direction of the absorbent articles is directed along the direction of transport, and the absorbent articles with the lateral direction thereof being along the direction of transport are transported toward the gathering position where the absorbent articles and the continuous sheet are gathered.

According to such a method for manufacturing a package of an absorbent article, the absorbent articles are transported to the gathering position at which the absorbent articles and the continuous sheet are gathered, with the lateral direction of the absorbent article being along the direction of transport. Accordingly, the pitch of the absorbent articles in the direction of transport can be reduced, and this can make it possible to reduce the length in the direction of transport of the continuous sheet necessary for a single package. Thus, according to an amount corresponding to such reduction in length, positioning accuracy when the absorbent articles are disposed on the continuous sheet can be enhanced.

In such a method for manufacturing a package of an absorbent article, it is preferable that in the first transport process, a pitch changing device is used to receive the absorbent articles transported from the upstream side in the direction of transport at a transport pitch different from the first pitch, and the pitch changing device changes the transport pitch of the received absorbent articles into the first pitch, to transport the absorbent articles in the direction of transport.

According to such a method for manufacturing a package of an absorbent article, since the transport pitch is changed into the first pitch, it becomes easier to dispose the absorbent article at the appropriate position on the continuous sheet.

In such a method for manufacturing a package of an absorbent article, it is preferable that in the state-of-stretch adjusting process, a state of stretch of the continuous sheet is adjusted using the state-of-stretch adjusting device also based on the information on the distance of the position.

According to such a method for manufacturing a package of an absorbent article, it becomes possible that adjustment of the state of stretch made by the state-of-stretch adjusting device is substantially prevented from being affected by adjustment of the transport speed value made by the delivering device. Accordingly, it becomes possible to more reliably make an adjustment such that the center-to-center interval between the mark elements will be the first pitch.

Further, an apparatus for manufacturing a package formed by wrapping an absorbent article with a packaging sheet, the apparatus comprising: a first transporting apparatus for transporting a plurality of absorbent articles at a first pitch; a second transporting apparatus for transporting a continuous sheet of packaging sheets in a direction of transport toward a gathering position, the gathering position being a position where the continuous sheet and the absorbent articles are gathered, the continuous sheet having mark elements wherein the mark elements are aligned in the direction of transport at a center-to-center interval that is smaller than the first pitch when the continuous sheet is in an unstretched state; and a joining device for joining each of the absorbent articles to the continuous sheet at the gathering position, the second transporting apparatus comprising a state-of-stretch adjusting device for acquiring information on a difference of the center-to-center interval between the mark elements from the first pitch, and adjusting a state of stretch of the continuous sheet based on the information on the difference of the center-to-center interval; and a delivering device for acquiring information on a distance of a position of one of the mark elements from a reference position, the reference position being based on the first pitch on the continuous sheet, and adjusting, based on the information on the distance of the position, a transport speed value at a time when delivering the continuous sheet toward the gathering position.

According to such an apparatus of manufacturing a package of an absorbent article, the functions and effects as in the case of the aforementioned manufacturing method can be achieved.

### =Present Embodiment=

A method and apparatus for manufacturing a package of an absorbent article according to an embodiment of the present disclosure is used in a manufacturing line LM for a package 7p of a sanitary napkin 1. That is, in an embodiment of the present disclosure, the package 7p of the sanitary napkin 1 serving as an absorbent article is manufactured on the basis of the manufacturing method and manufacturing apparatus.

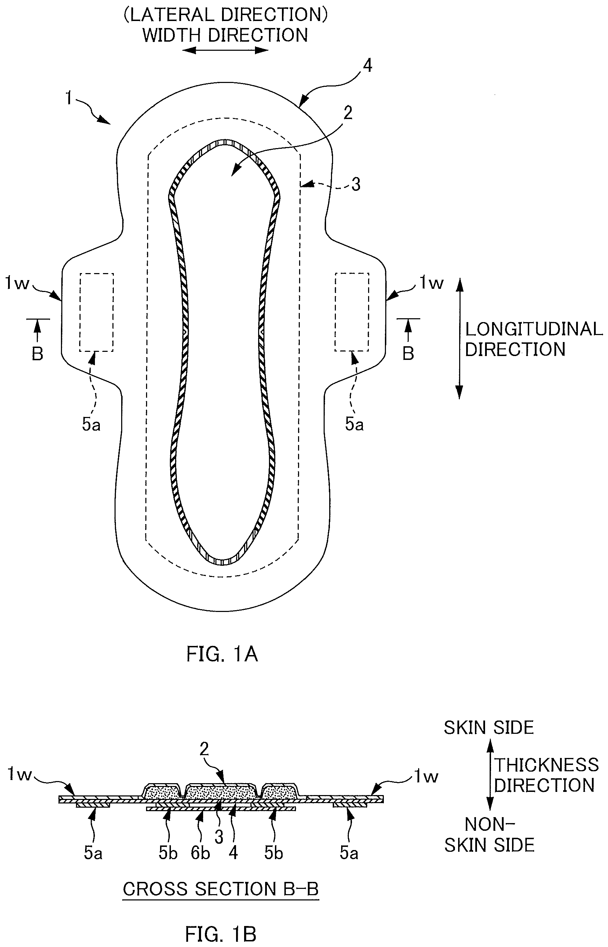

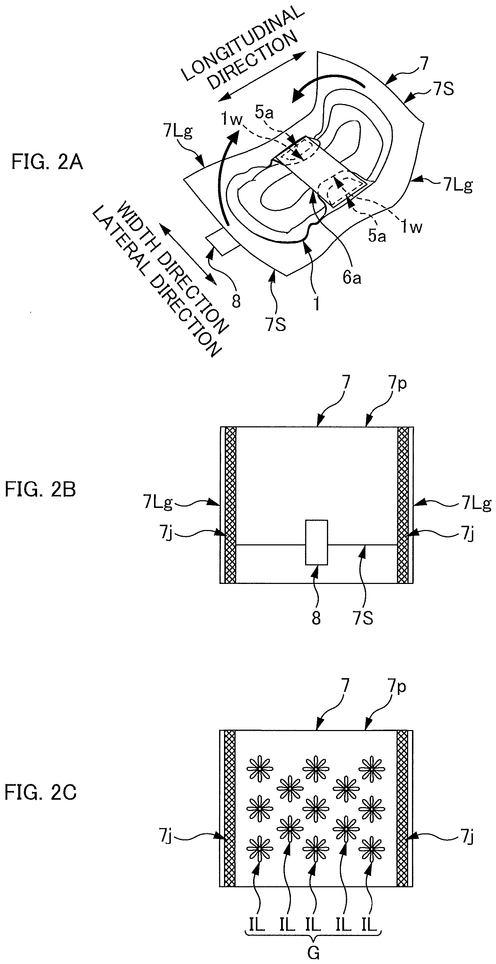

FIG. 1A is a schematic plan view illustrating the napkin 1, and FIG. 1B is a cross sectional view taken along line B-B of FIG. 1A. FIG. 2A is a schematic perspective view illustrating the napkin 1 placed on a packaging sheet 7, FIG. 2B is a schematic plan view illustrating the package 7p obtained by wrapping the napkin 1 with the packaging sheet 7, and FIG. 2C is a schematic plan view illustrating the package 7p when viewed from the side opposite to the side in FIG. 2B.

As illustrated in FIGS. 1A and 1B, the napkin 1 includes, for example, a liquid permeable top sheet 2, such as a nonwoven fabric, a liquid-impermeable back sheet 4, such as a film, and an absorbent body 3, such as pulp fibers, which is disposed between the top sheet 2 and the back sheet 4 to absorb discharges from a human body. These sheets 2, 4 are affixed to each other in a portion extending outward from the absorbent body 3 such that the absorbent body 3 is held between these sheets 2, 4.

The planar shape of the napkin 1 is a substantially rectangular shape having a longitudinal direction and a width direction (hereinafter, also referred to as the lateral direction), but may have wing portions 1w, 1w in a pair respectively protruding outward in the width direction at substantially central positions in the longitudinal direction in the napkin 1 in some cases. This embodiment has such a configuration.

When the napkin 1 is used, these wing portions 1w, 1w are folded back to the back sheet 4 side to sandwich an undergarment in cooperation with the back sheet 4 and bonded and fixed to the undergarment. Thus, adhesives 5a, 5a are applied to surfaces, of the wing portions 1w, 1w, which are to contact the undergarment when the wing portions 1w, 1w are folded back. Adhesives 5b, 5b for the same purpose are applied to a surface of the back sheet 4 which is to contact the undergarment when the napkin 1 is used.

In order to preserve such adhesive strength of the adhesives 5b, 5b until the time when the napkin 1 is used, as illustrated in FIGS. 1B and 2A, protection sheets 6a, 6b are provided to cover such aforementioned regions where the adhesives 5b, 5b have been applied. In specific, before use, the wing portions 1w, 1w in a pair are protected by the single protection sheet 6a spanning the wing portions 1w, 1w that have been folded back to the top sheet 2 side (FIG. 2A). On the other hand, the adhesives 5b, 5b are also protected by the protection sheet 6b provided to cover substantially all the surfaces of the regions where the adhesives 5b, 5b have been applied in the back sheet 4 (FIG. 1B).

At such a time before use, the napkin 1 is in the form of the package 7p having been individually packaged with the packaging sheet 7 as illustrated in FIGS. 2A and 2B. That is, first, as illustrated in FIG. 2A, the napkin 1 that is placed on the substantially rectangular-shaped single-sheet packaging sheet 7 having a planar size larger than that of the napkin 1, with their longitudinal directions aligned with each other, is tri-folded in the longitudinal direction together with the packaging sheet 7. Then, in such a tri-folded state of FIG. 2B, a shorter side 7S of the packaging sheet 7 is fixed to the same packaging sheet 7 with an adhesive of a tape member 8 while, in a pair of longer sides 7Lg, 7Lg of the packaging sheet 7, portions facing each other of the packaging sheet 7 are welded to form sealed portions 7j, 7j. In the aforementioned placed state, the protection sheet 6b of the napkin 1 may adhere to be fixed to the packaging sheet 7 at a plurality of positions and, in this example, the protection sheet 6b of the napkin 1 adheres to the packaging sheet 7 at three positions in the longitudinal direction of the packaging sheet 7.

A material of such a packaging sheet 7 may include, for example, a thermoplastic resin film, such as polyethylene (PE) and polypropylene (PP), a nonwoven fabric containing thermoplastic resin fibers, such as PE fibers and PP fibers, and a PE film is used herein, however, it is not limited thereto as long as it has a predetermined stretchability.

Further, as illustrated in FIG. 2C, a flower pattern G is printed onto the packaging sheet 7 as an example of a pattern for design use. In specific, the pattern G includes a plurality of flower illustrations IL, IL ..., and these flower illustrations IL, IL ... are combined to form the single pattern G. Accordingly, the flower pattern G is printed across the substantially entire surface on one surface of the package 7p. Further, a printing method of the pattern G includes, for example, gravure printing, flexographic printing, and ink jet printing.

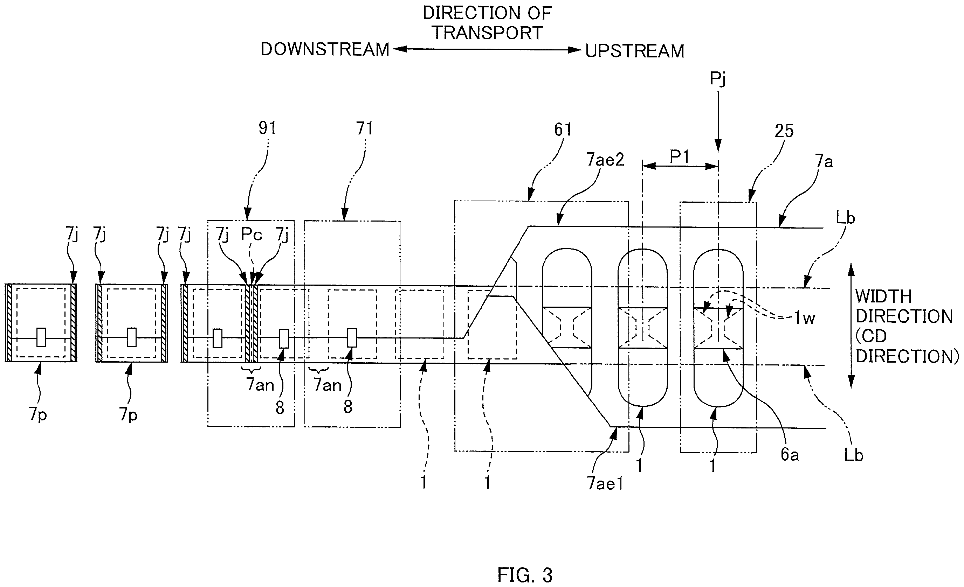

FIG. 3 is a schematic plan view illustrating a process of forming the package 7p of the napkin 1.

As illustrated in FIG. 3, first, the packaging sheet 7 is transported in the direction of transport in the form of a continuous sheet 7a that is along the direction of transport. Then, the napkins 1 that are transported while being aligned at product pitches P1 are gathered with the packaging sheet 7 at a gathering position Pj in the direction of transport. Note that, at this point, the napkin 1 is in a finished form, that is, the wing portions 1w, 1w are folded back as in FIG. 2A, with the aforementioned protection sheets 6a, 6b having been attached thereto. Further, as illustrated in FIG. 3, the napkins 1 are transported with their width direction (lateral direction) being directed in the direction of transport, i.e., in a lateral flow.

Then, at this gathering position Pj, the napkins 1, 1 are placed, by a joining device 25 that is positioned at the position Pj, in the direction of transport at the product pitches P1 on an upper surface which is one side of the surfaces of the continuous sheet 7a of the packaging sheet, so that the napkins 1 are fixed to the continuous sheet 7a with an adhesive (not shown).

Thereafter, the continuous sheet 7a on which the napkins 1 are placed passes through the position of a folding device 61 in the direction of transport, and when passing therethrough, the continuous sheet 7a is tri-folded in the width direction by the device 61. That is, each of one end 7ae1 and the other end 7ae2 in the width direction of the continuous sheet 7a is folded inward in the width direction. At this time, the napkin 1 is also tri-folded together with the continuous sheet 7a.

Then, the tri-folded continuous sheet 7a passes through the position of a tape-member attaching device 71 in the direction of transport, and when passing therethrough, the tape member 8 is attached to the continuous sheet 7a by the device 71 so as to straddle the aforementioned ends 7ae1, 7ae2 overlapping in the thickness direction. Then, the continuous sheet 7a is fixed in the tri-folded state with the tape member 8.

Thereafter, the tri-folded continuous sheet 7a passes through the position of a sealing cutting device 91 in the direction of transport and, when passing therethrough, the device 91 performs heat welding to a part 7an corresponding to a part of the continuous sheet 7a between the napkins 1, 1 adjacent to each other in the direction of transport as illustrated in FIG. 3, in other words, the napkin absent part 7an where the napkin 1 does not exist in the sheet 7a. Accordingly, parts of the continuous sheet 7a overlapping in thickness direction are joined together by welding to form a pair of sealed portions 7j, 7j side by side. Further, at this time, the continuous sheet 7a is cut into two, the front and the rear in the direction of transport, at a position Pc between a pair of sealed portions 7j, 7j by the device 91, so that the package 7p of the napkin 1 is formed.

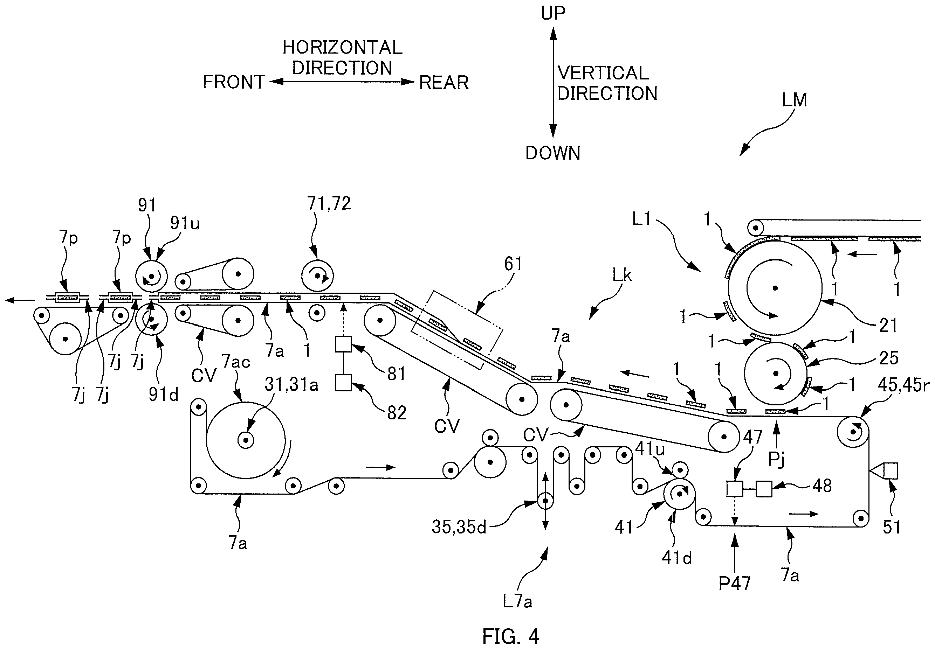

FIG. 4 is a schematic side view illustrating the manufacturing line LM for the package 7p of the napkin 1.

The manufacturing line LM includes: a napkin feeding system L1 (corresponding to a first transporting apparatus) to feed the napkins 1; a packaging sheet feeding system L7a (corresponding to a second transporting apparatus) to feed the continuous sheet 7a of the packaging sheet; and a processing system Lk to perform appropriate processing/treatment to the continuous sheet 7a of the packaging sheet to which the napkins 1 are joined. Then, the napkin feeding system L1 and the packaging sheet feeding system L7a gather at the predetermined gathering position Pj as well as are connected to the processing system Lk downstream of the position Pj.

Note that, in each of the systems L1, L7a, Lk, an appropriate transporting apparatus such as a belt conveyor or conveyor rollers are arranged. Thus, when there is no particular explanation, it is assumed that an object to be transported such as the continuous sheet 7a or the napkins 1 are transported in the direction of transport by such a transporting apparatus. The belt conveyor includes: for example, a common belt conveyor including an endless belt configured to be driven to circulate as a transport surface; a suction belt conveyor having a suction function in an outer circumferential surface of an endless belt; and the like.

Further, in this manufacturing line LM, a CD direction (a direction of penetrating the paper in FIG. 4) is defined as the width direction in the line LM. In this example, the CD direction is directed in the horizontal direction, but it is not limited thereto. Further, in this example, an up-and-down direction that is vertical and a fore-and-aft direction that is horizontal are defined as directions orthogonal to the CD direction, and accordingly the directions of transport of the napkins 1 and the continuous sheet 7a of the packaging sheet are defined by both the up-and-down direction and the fore-and-aft direction, depending on positions in the directions of transport. The width direction of the continuous sheet 7a is parallel to the CD direction. Further, when a direction orthogonal to the CD direction and the direction of transport is defined as a Z direction, the Z direction is parallel to the thickness directions of the continuous sheet 7a and the napkin 1.

### Napkin Feeding System L1

The napkin feeding system L1 includes: a state-of-napkin changing device 21 for receiving the finished napkin 1 that is transported from an upstream process and transporting the napkin 1 while changing the state thereof into the state suitable for packaging; and the joining device 25 that is disposed at the aforementioned gathering position Pj to join the napkins 1 to the continuous sheet 7a of the packaging sheet.

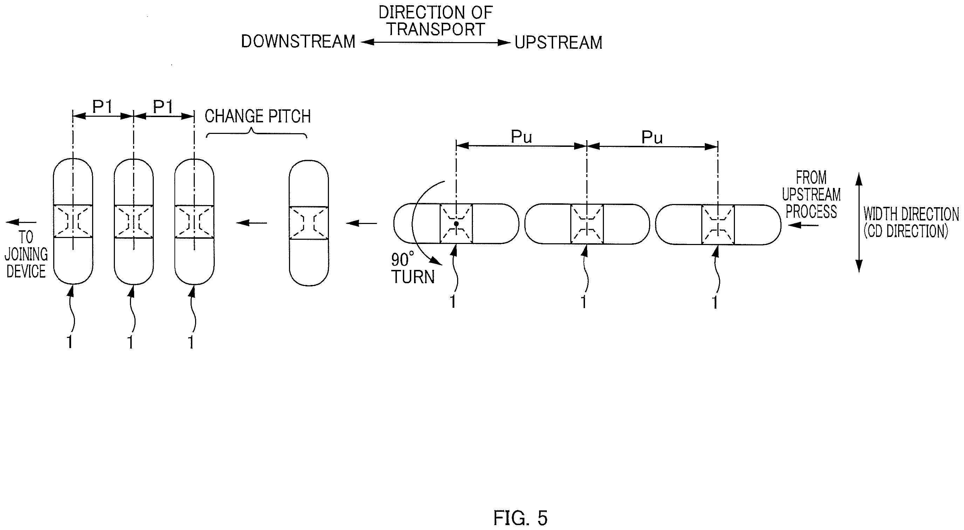

As illustrated in a schematic diagram in FIG. 5, the state-of-napkin changing device 21 (corresponding to a direction changing device and a pitch changing device), the napkins 1 are delivered in the transport form of a so-called longitudinal flow from the upstream process. That is, the napkins 1 are delivered with their longitudinal direction being directed in the direction of transport. Thus, in the device 21, first, the form of transport of the napkins 1 is changed from the longitudinal flow into the lateral flow, which is suitable for packaging. That is, the direction of each of the napkins 1 is changed by 90 degrees, so that the longitudinal direction of the napkins 1 is directed in the CD direction.

Further, as illustrated in FIG. 5, the napkins 1 are delivered from the upstream process to the device 21 at transport pitches Pu, which are different from the product pitches P1 (corresponding to a first pitch). Accordingly, the transport pitches Pu of the napkins 1 are changed into the product pitches P1 by pitch-changing. Then, such napkins 1 that are in a lateral flow and aligned at the product pitches P1 are delivered to the joining device 25 that is located downstream in the direction of transport.

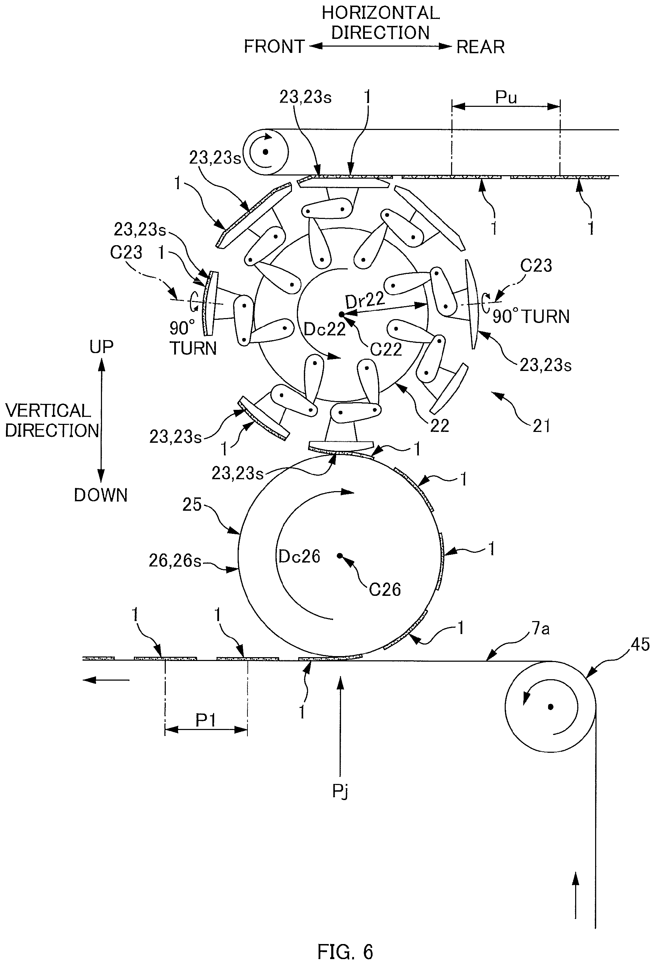

For example, the following device 21 can be used for the state-of-napkin changing device 21 that has such a direction changing function and a pitch changing function. FIG. 6 is a schematic side view of this device 21.

This device 21 includes a rotating drum 22. The rotating drum 22 is driven to rotate about a rotational axis C22 that is along the CD direction. Further, on an outer periphery of the rotating drum 22, a plurality of holding pads 23, 23 ... are provided to be aligned in the direction of rotation Dc22 of the rotating drum 22. Then, each of the holding pads 23 includes a holding surface 23s directed outward in a direction of rotational radius Dr22 of the rotating drum 22 and is capable of receiving the napkin 1 by suction and holding it with this holding surface 23s. Further, the holding pad 23 is turnable about an axis C23 that is along the direction of rotational radius Dr22 penetrating the center of a plane of the holding surface 23s, thereby achieving a direction changing function. That is, the napkin 1 is turned by 90 degrees about this axis C23 so that the longitudinal direction of the napkin 1 can be changed from the direction of transport which is the direction of rotation Dc22 into the CD direction. Furthermore, the holding pad 23 is configured to be movable in the direction of rotation Dc22 relative to the rotating drum 22 in conjunction with the rotational operation based on a cam mechanism, etc., while moving in the direction of rotation Dc22 together with the rotational operation of the rotating drum 22, thereby implementing a pitch changing function. In other words, by changing a pitch between the holding pads 23, 23 adjacent to each other in the direction of rotation Dc22, the transport pitches Pu, which are different from the product pitches P1, when the napkins 1 are received can be changed into the product pitches P1.

The joining device 25 includes: a rotating drum 26 capable of rotating about a rotational axis C26 that is along the CD direction, and a servo motor (not shown) serving as a power source to drive the rotating drum 26. Further, in an outer circumferential surface 26s of the rotating drum 26, a suction force is generated by suction. Thus, the outer circumferential surface 26s receives and holds the napkins 1 delivered in the direction of transport at the product pitches P1 from the state-of-napkin changing device 21. Thereafter, the rotating drum 26 is driven to rotate to transport the napkins 1 at the product pitches P1 with a direction of rotation Dc26 being set as the direction of transport, and then each of the napkins 1 is pressed against an upper surface which is one surface of the continuous sheet 7a at the gathering position Pj, so that the napkins 1 are joined to the continuous sheet 7a with an adhesive (corresponding to a joining process).

Here, the rotating drum 26 is driven to rotate based on synchronization signals. The synchronization signals are obtained by outputting signals per unit repeatedly, and the signals per unit are rotational angle signals constituted corresponding to rotational angle values of 0 to 360 degrees. Further, the devices 71, 91 of the processing system Lk, which will be described later, each have a patterned process operations per unit to be repeated for each of the napkins 1, such process operations per unit are associated with the signals per unit in one-to-one correspondence under position control. In this joining device 25, a transport operation at the product pitch P1 is associated with a unit of process operations. That is, such a synchronization signal is transmitted to a servo motor of the rotating drum 26, as well as position-control is performed such that the rotating drum 26 is rotated by a rotation angle corresponding to the product pitch P1 in response to each synchronization signal corresponding to signals per unit. Therefore, every time when such a unit of signals is output, the rotating drum 26 transports the napkin 1 in the direction of transport by the product pitch P1.

The synchronization signals are generated in a suitable control unit (not shown) such as a sequencer or a computer. That is, the control unit includes a processor and a memory, and the memory stores a program for generating the synchronization signals in advance. Then, the processor reads the program from the memory and executes the program to repeatedly generate a unit of signals of the synchronization signal. However, the generation of the signals is not limited thereto. For example, the synchronization signals may be produced by an electric circuit, or the synchronization signals may be produced by detecting, with a suitable detector, process operations per unit that are repeatedly performed onto each of the napkins 1 by another key device. For example, a rotary die cutter (not shown) for performing punching to form the napkin 1 may be provided with a rotary encoder (not shown) for detecting its rotation operation, and this encoder outputs a unit of signals every time one single napkin is formed by punching, in cooperation with a rotation operation of the cutter roll.

### Packaging Sheet Feeding System L7a

As illustrated in FIG. 4, the packaging sheet feeding system L7a includes: a feeding device 31 for feeding the continuous sheet 7a of the packaging sheet from a material coil 7ac; and a delivering device 45 for delivering the continuous sheet 7a of the packaging sheet that is fed to be transported toward the gathering position Pj, at which the continuous sheet 7a is gathered with the napkins 1 on the napkin feeding system L1. Then, the napkins 1 are placed on and joined to the continuous sheet 7a of the packaging sheet at the gathering position Pj by the aforementioned joining device 25.

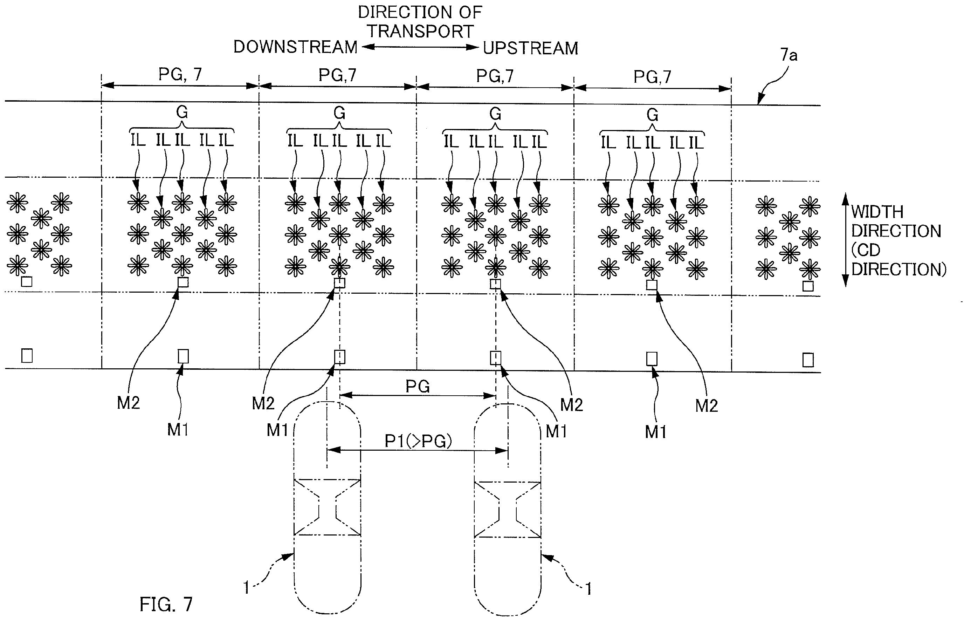

As can be understood from the feeding device 31 being used, the continuous sheet 7a, which is a material of the packaging sheet 7, is carried onto the manufacturing line LM in the form of the material coil 7ac. The material coil 7ac means herein one obtained by winding up the continuous sheet 7a of the packaging sheet in the direction of continuation. Before the continuous sheet 7a of the packaging sheet is formed in the form of such a material coil 7ac, the aforementioned patterns G, G ... are printed thereon at predetermined print pitches PG in the direction of continuation in advance in a separate factory or the like, as illustrated in a schematic plan view in FIG. 7. The print pitches PG are smaller than the product pitches P1 of the napkins 1.

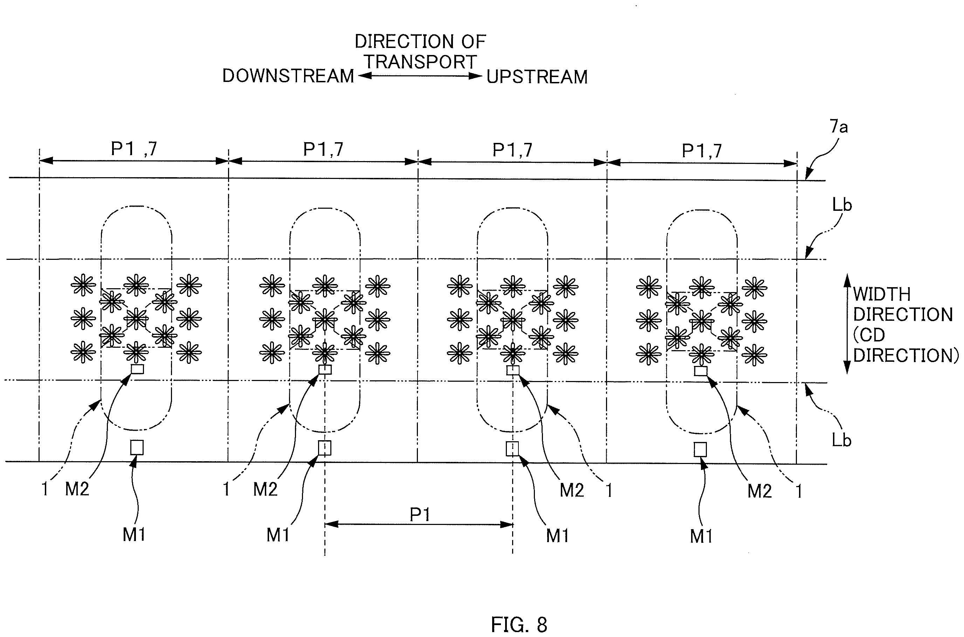

Thus, in an embodiment of the present disclosure, prior to reaching the gathering position Pj, the continuous sheet 7a of the packaging sheet is stretched in the direction of transport. That is, basically, the napkins 1 are placed on and joined to the continuous sheet 7a of the packaging sheet that have been stretched at a target rate of stretch Ms(=P1/PG) obtained by dividing the product pitch P1 by the print pitch PG. Accordingly, as illustrated in a schematic plan view in FIG. 8, the napkins 1 are arranged at appropriate positions corresponding to the patterns G on the continuous sheet 7a of the packaging sheet. The process of changing the state of the sheet into that having been stretched at the target rate of stretch is performed by a state-of-stretch adjusting device 41. The device 41 is disposed in a position between the feeding device 31 and the delivering device 45 on a transport path of the continuous sheet 7a of the packaging sheet, as illustrated in FIG. 4.

Note that the rate of stretch means herein the rate at which a natural length Ln in a non-stretched state (a length in a state where no external force is applied) has been stretched to be the total length Lt in the direction of continuation (direction of transport) of the stretched continuous sheet 7a, and the rate can be obtained, for example, by dividing the total stretched length Lt by the natural length Ln, as a division value (=Lt/Ln). Furthermore, in this example, when the print pitch PG is set to "1", for example, the product pitch P1 is set to "1.026", and thus the target rate of stretch Ms of the continuous sheet 7a of the packaging sheet at the gathering position Pj results in 1.026. However, it is not limited thereto.

The devices 31, 41, 45 related to the packaging sheet feeding system L7a will be described below.

### (1) Feeding Device 31

As illustrated in FIG. 4, the feeding device 31 includes: a rotational axis portion 31a along the CD direction, a servo motor (not shown) serving as a power source to drive the rotational axis portion 31a to rotate; a tension controller 35 to adjust the tension of the continuous sheet 7a of the packaging sheet fed to be transported from the rotational axis portion 31a. Then, the material coil 7ac is driven to rotate based on the above tension value, while the rotational axis portion 31a is inserted into a through hole at the central portion of the material coil 7ac and supports the material coil 7ac, so that the continuous sheet 7a of the packaging sheet is fed from the material coil 7ac.

The rotational axis portion 31a is a shaft member, for example, along the CD direction. Then, the rotational axis portion 31a is rotatably supported by an appropriate bearing member (not shown) provided on the manufacturing line IM.

The tension controller 35 of a dancer type is used. That is, the controller 35 includes: a dancer roll 35d that is rotatable about a rotational axis along the CD direction while being movably guided in a predetermined direction (up-and-down direction in FIG. 4); a measuring instrument (not shown), such as an encoder, for detecting a position in the aforementioned predetermined direction of the dancer roll 35d and outputting such positional information; a control unit (not shown), such as a sequencer or a computer, to which the aforementioned positional information is input. Then, the continuous sheet 7a of the packaging sheet is wrapped around the dancer roll 35d so that a loop of the continuous sheet 7a is formed, and the control unit controls a feeding speed (mpm) of the rotational axis portion 31a so that the size of the loop is constant based on the input positional information. Accordingly, adjustment is made such that the tension of the continuous sheet 7a at the position of the dancer roll 35d is a predetermined target value.

### (2) State-of-stretch Adjusting Device 41 and Delivering device 45

As illustrated in FIG. 4, the state-of-stretch adjusting device 41 includes: a pair of upper and lower nip rolls 41u, 41d to rotate around rotational axes along the CD direction; and a servo motor (not shown) serving as a power source to drive the pair of nip rolls 41u, 41d to rotate. Further, the delivering device 45 located downstream of the state-of-stretch adjusting device 41 includes: a roll 45r to rotate around a rotational axis that is along the CD direction, and a servo motor (not shown) serving as a power source to drive the roll 45r to rotate.

Then, the delivering device 45 delivers the continuous sheet 7a of the packaging sheet toward the gathering position Pj, and when delivering this sheet, the transport speed value (mpm) of the continuous sheet 7a is adjusted such that the patterns G are respectively positioned at reference positions on the continuous sheet 7a of the packaging sheet that are determined based on the product pitches P1 (corresponding to a transport speed value adjusting process). That is, the transport speed value of the continuous sheet 7a of the packaging sheet is adjusted such that the patterns G are positioned at reference positions indicated by the aforementioned synchronization signals, respectively. Accordingly, at the gathering position Pj, the napkins 1 are respectively placed and joined at appropriate positions corresponding to the patterns G of the continuous sheet 7a, as illustrated in FIG. 8.

On the other hand, the state-of-stretch adjusting device 41 adjusts the state of stretch of the continuous sheet 7a in cooperation with the delivering device 45 (corresponding to a state-of-stretch adjusting process). That is, stretch is adjusted such that the rate of the stretch of the continuous sheet 7a is the target rate of stretch Ms. Accordingly, pitches at which the patterns G are arranged in the direction of transport are adjusted to be matched with the product pitches P1 (FIG. 8). This also effectively contributes to the placement of the napkins 1 at the appropriate positions.

Such registration at the appropriate positions and adjustment of the rate of stretch is implemented as follows using an imaging device 47, such as a CCD camera, and a control unit 48, such as a computer, illustrated in FIG. 4.

First, as illustrated in FIG. 7, substantially rectangular registration marks M1 (corresponding to mark elements) are printed at the print pitches PG corresponding to the patterns G on the continuous sheet 7a of the packaging sheet, in advance in a separate factory. Further, as illustrated in FIG. 4, the imaging device 47 images the continuous sheet 7a at a position P47 (hereinafter also referred to as an imaging position P47) between the state-of-stretch adjusting device 41 and the delivering device 45 in the direction of transport, to produce image data of the continuous sheet 7a, and here the imaging device 47 receives the synchronization signals. Thus, the continuous sheet 7a is imaged every time the rotational angle value of the synchronization signal is matched with the defined rotational angle value recorded beforehand in the memory of the imaging device 47. The imaging is performed in a field of view (angle of view) in which at least two registration marks M1, M1 adjacent to each other in the direction of transport are within a captured image.

Then, the control unit 48 acquires information on the distance of one of the registration marks M1 from a reference position on the continuous sheet 7a of the packaging sheet, by analyzing image data transmitted from the imaging device 47 through a binarization process or the like. For example, the position of the registration mark M1 on the image data is acquired through the binarization process or the like, and the distance between this position and the reference position on the image data, which is recorded beforehand in the memory of the control unit 48, is calculated, and the result is taken as information on the distance of this position.

Then, based on the information on the distance of a position, a transport speed value when the delivering device 45 delivers the continuous sheet 7a toward the gathering position Pj is adjusted. For example, the control unit 48 controls the transport speed value such that, when the aforementioned information on the distance indicates that the "registration mark M1 is positioned downstream in the direction of transport from the reference position", the transport speed value of the roll 45r in the delivering device 45 is made smaller than the current value, by the amount of adjustment obtained by multiplying the distance by a predetermined gain, and when the information indicates that the "registration mark M1 is positioned upstream in the direction of transport from the reference position", the transport speed value is made larger than the current value by the amount of adjustment obtained by multiplying the distance by a predetermined gain.

Further, the control unit 48 analyzes the same image data to acquire information on the difference between the center-to-center interval of the registration marks M1 and the product pitch P1. For example, the center-to-center interval of the registration marks M1, M1 adjacent to each other in the direction of transport is obtained on the image data, based on the position of the registration mark M1 on the image data, to calculate a difference between the distance of this interval and the value of the product pitch P1 stored beforehand in the memory of the control unit 48, and this difference is defined as information on the difference of the aforementioned center-to-center interval. Then, the state of stretch of the continuous sheet 7a is adjusted based on the information on the difference of the center-to-center interval. For example, the control unit 48 controls the transport speed value such that, when the aforementioned information on the difference indicates that "the center-to-center interval is smaller than the product pitch P1", the transport speed value of the nip rolls 41u, 41d of the state-of-stretch adjusting device 41 is made smaller than the current value, by the amount of adjustment obtained by multiplying the difference by the predetermined gain, thereby stretching the continuous sheet 7a and, on the other hand, when the information indicates that "the center-to-center interval is larger than the product pitch P1", the transport speed value of the nip rolls 41u, 41d is made larger than the current value by the amount of adjustment obtained by multiplying the difference by the predetermined gain, thereby contracting the continuous sheet 7a.

It should be noted that in association with an adjustment of the transport speed value by the delivering device 45, the state of stretch of the continuous sheet 7a between the delivering device 45 and the state-of-stretch adjusting device 41 may change by the amount of adjustment. Thus, it is preferable not only to adjust the transport speed value of the delivering device 45 but to change the transport speed value of the state-of-stretch adjusting device 41 similarly based on the information on the distance of the position, and in this example, the transport speed value is changed similarly as such. Accordingly, it becomes possible to substantially prevent the adjustment of the transport speed value made by the delivering device 45 from affecting the adjustment of the stretch made by the state-of-stretch adjusting device 41.

As illustrated in FIG. 4, in this example, an adhesive for joining the napkin 1 onto the continuous sheet 7a is applied on the continuous sheet 7a by an adhesive applying device 51. Then, such adhesives are applied with discontinuous connection in response to each unit of signals based on the synchronization signal. Thus, it is possible to accurately apply the adhesives to the positions, at which the napkins 1 are to be placed on the continuous sheet 7a.

Further, in this example, the position in the direction of transport at which the device 51 applies the adhesive to the continuous sheet 7a is set between the delivering device 45 and the imaging position P47 of the imaging device 47. Thus, the adhesive is applied onto the continuous sheet 7a at the position relatively closer to the gathering position Pj. This makes it possible to reduce the change in position of the adhesive in the direction of transport after the application of the adhesive. Accordingly, the napkins 1 can be reliably joined to the continuous sheet 7a.

### Processing System Lk

As illustrated in FIG. 4, in the processing system Lk, the continuous sheet 7a of the packaging sheet to which the napkins 1 are joined is transported in the direction of transport. Then, in the processing system Lk, the folding device 61, the tape-member attaching device 71, and the sealing cutting device 91 are arranged to be aligned in the direction of transport in this order.

The folding device 61 tri-folds, together with the napkins 1, the continuous sheet 7a of the packaging sheet to which the napkins 1 are joined (corresponding to a tri-folding process). The tape-member attaching device 71 attaches the tape members 8 (FIG. 2B, FIG. 3) to the continuous sheet 7a corresponding to the napkins 1 so that the continuous sheet 7a is fixed in the tri-folded state (corresponding to a fixing process). The sealing cutting device 91 forms a pair of the sealed portions 7j, 7j (FIG. 3) in the tri-folded continuous sheet 7a, as well as cuts the continuous sheet 7a at a position between the pair of the sealed portions 7j, 7j, thereby producing the packages 7p (corresponding to a forming process). Note that the tape-member attaching device 71 and the sealing cutting device 91 are driven fundamentally based on the synchronization signals.

Hereinafter, the devices 61, 71, 91 will be described.

### (1) Folding Device 61

The folding device 61 includes a folding member (not shown) such as an appropriate plate member or roller member on both sides in the CD direction. Then, as illustrated in FIGS. 3 and 4, when the continuous sheet 7a of the packaging sheet to which the napkins 1 are joined passes through the position of the folding member, the folding member contacts the continuous sheet 7a thereby folding both lateral ends 7ae1, 7ae2 of the continuous sheet 7a inward in the width direction together with the napkins 1. Accordingly, the continuous sheet 7a is made in a tri-folded state together with the napkins 1.

### (2) Tape-member Attaching Device 71

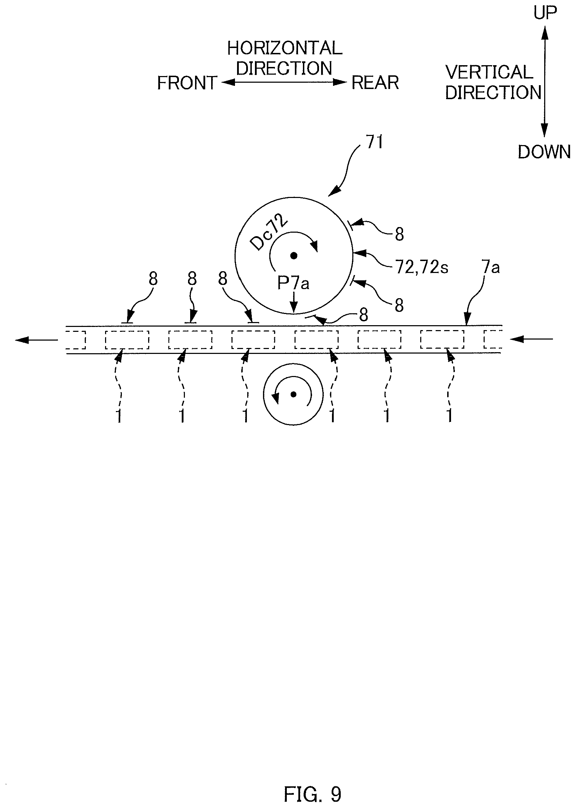

FIG. 9 is a schematic side view illustrating the tape-member attaching device 71.

The tape-member attaching device 71 includes: a rotating drum 72 (corresponding to a rotating body) that is rotatable about a rotational axis that is along the CD direction while an outer circumferential surface 72s thereof faces the transport path of the tri-folded continuous sheet 7a; a servo motor (not shown) serving as a power source for driving the rotating drum 72 to rotate. Then, a suction force is generated by suction in the outer circumferential surface 72s of the rotating drum 72, so that a plurality of the tape members 8, 8 ... are held onto the outer circumferential surface 72s to be aligned at the product pitches P1 in a direction of rotation Dc72. Then, when the tape members 8 are delivered to a facing position P7a (corresponding to a predetermined position) at which the tape members 8 face the tri-folded continuous sheet 7a by rotation of the rotating drum 72 and passes through the position P7a, the tape members 8 adhere to the continuous sheet 7a with adhesives of the tape members 8, thereby being delivered to the continuous sheet 7a from the outer circumferential surface 72s of the rotating drum 72.

The rotating drum 72 is rotated fundamentally based on the synchronization signal. That is, a servo motor of the rotating drum 72 is position-controlled such that the position where the tape member 8 is suctioned and held on the outer circumferential surface 72s will be in the instructed position in the direction of rotation Dc72 indicated by the synchronization signal. Thus, basically, each time when the unit of signals of the synchronization signal is inputted, the tape members 8 that are arranged at the product pitches P1 on the outer circumferential surface 72s are transported in the direction of rotation Dc72 by the product pitch P1.

Incidentally, the speed of a transporting apparatus CV (FIG. 4) such as a belt conveyor or conveyor rollers for transporting the continuous sheet 7a in the processing system Lk is controlled basically with a transport speed value corresponding to the synchronization signal being used as an instruction value. However, the transport speed value may change, for example, substantially periodically due to various disturbances under control and/or variations in stretchability of the continuous sheet 7a itself.

In such a case, the stretched state of the tri-folded continuous sheet 7a can change. As a result, the aforementioned state where the continuous sheet 7a has been stretched at the target rate of stretch Ms changes. Then, at a position upstream in the direction of transport from the rotating drum 72, the pitches in the direction of transport of the patterns G on the continuous sheet 7a may be displaced from the product pitches P1, and similarly, the pitches at which the napkins 1 are arranged may also be displaced from the product pitches P1. In such a case, even if trying to bond the tape member 8 to the target position (appropriate position) on the continuous sheet 7a based on the synchronization signal, the tape member 8 may be deviated from the target position.

Accordingly, in order to deal with such an issue, the following is performed in this example. That is, first, specific marks M2, which are printed into a rectangular shape on the continuous sheet 7a separately from the aforementioned registration marks M1, are referred to as signs M2, as illustrated in FIG. 8. Then, information on the distance of the position in the direction of transport of such a sign M2 from a reference position, at which the sign M2 is to be originally positioned when the continuous sheet 7a is maintained at the product pitches P1, is acquired (corresponding to a second position distance information acquisition process), as well as the timing in which the rotating drum 72 delivers the tape member 8 to the aforementioned facing position P7a is shifted from the timing indicated by the synchronization signal, based on the information on the distance of this position.

Such timing shift is implemented by using an imaging device 81, such as a CCD camera, and a control unit 82 (FIG. 4), such as a computer, as in the case of the aforementioned packaging sheet feeding system L7a as follows.

First, as illustrated in FIG. 7, the marks M2 (corresponding to second mark elements), which should be the aforementioned signs M2, are printed on the continuous sheet 7a at the aforementioned print pitches PG in advance in a separate factory. Then, after passing the gathering position Pj, the signs M2 are positioned corresponding to the napkins 1, as illustrated in FIG. 8. Further, the imaging device 81 captures an image of the continuous sheet 7a, in the position between the folding device 61 and the rotating drum 72 of the tape-member attaching device 71 in the direction of transport, to produce image data of the continuous sheet, and here the imaging device 81 receives the synchronization signals. Thus, the continuous sheet 7a is imaged each time when the rotational angle value of the synchronization signal is matched with the defined rotational angle value recorded beforehand in the memory of the imaging device 81. Such an imaging operation is performed in a field of view (angle of view) in which at least one sign M2 is captured within an image.

Then, the control unit 82 acquires information on the distance of the sign M2 from a reference position on the continuous sheet 7a, by analyzing the image data transmitted from the imaging device 81 through a binarization process or the like. The reference position means herein a position at which the aforementioned sign M2 is to be originally positioned when the continuous sheet 7a is maintained at the product pitches P1 as described above. Then, in this example, the information on the reference position on the image data is recorded beforehand in the memory of the control unit 82. Accordingly, for example, the position of the sign M2 on the image data is acquired through the binarization process or the like, as well as the distance of this position from the reference position on the aforementioned image data is calculated, and the result is taken as information on the distance of this position.

Then, based on the information on the distance of this position, the instructed position in the direction of rotation Dc72 indicated by the synchronization signal is adjusted. For example, the control unit 82 controls the instructed position such that, when the aforementioned information on the distance indicates that "the sign M2 is positioned downstream in the direction of transport from the reference position", the above-described instructed position is increased by the amount of adjustment obtained by multiplying the distance by a predetermined gain, and when the information indicates that "the sign M2 is positioned upstream in the direction of transport from the reference position", the instructed position is reduced by the amount of adjustment obtained by multiplying the distance by a predetermined gain.

In this example, the tri-folding process of the continuous sheet 7a illustrated in FIG. 3 is performed by folding both lateral ends 7ae1, 7ae2 inward along the folding lines Lb, Lb defined in two positions in the width direction (CD direction) in the continuous sheet 7a. In such a case, it is preferable that the sign M2 is provided between the folding lines Lb, Lb, as illustrated in FIG. 8. If the sign M2 is provided in such a position, folding in the tri-folding process is unlikely to affect the sign M2, and as a result, the sign M2 can be stably imaged by the imaging device 81.

Further, as another method for joining the tape member 8 to the target position (appropriate position) on the continuous sheet 7a, it can be also considered to use the method as in the delivering device 45. That is, it can be considered to adjust the state of stretch of the continuous sheet 7a based on the aforementioned information on the distance of the position. However, the napkin 1 has already been joined onto the continuous sheet 7a, in the position of the tape-member attaching device 71. Thus, at this point of time, it is difficult to smoothly change the state of stretch of the continuous sheet 7a, resulting in the tape-member attaching device 71 being controlled based on the information on the distance as described above, in this example. This also applies to the sealing cutting device 91 which will be described later.

### (3) Sealing Cutting Device 91

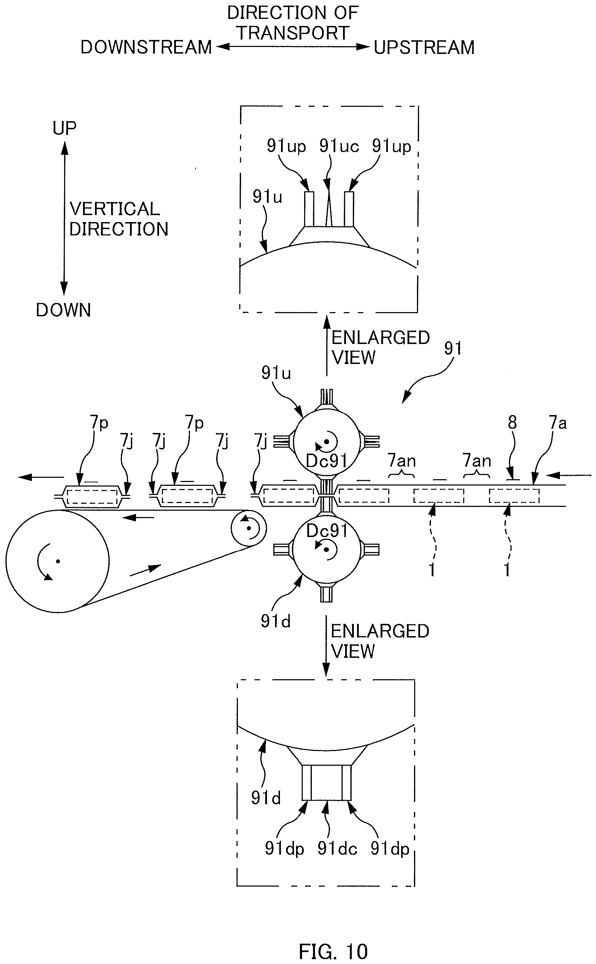

FIG. 10 is a schematic side view illustrating the sealing cutting device 91. In addition, FIG. 10 also illustrates a partial enlarged view illustrating the device 91.

As has described above, the sealing cutting device 91 forms a pair of the sealed portions 7j, 7j by heat sealing, at the part 7an between the napkins 1, 1 adjacent to each other in the direction of transport in the tri-folded continuous sheet 7a, that is, the napkin absent part 7an where the napkin 1 does not exist in the continuous sheet 7a, as well as the continuous sheet 7a is cut at a position between the pair of sealed portions 7j, 7j, to produce the packages 7p of the napkins 1.

Such a sealing cutting device 91 includes: a pair of upper and lower rolls 91u, 91d that are rotatable about rotational axes that are along the CD direction; and a servo motor (not shown) serving as a power source to drive the pair of rolls 91u, 91d to rotate.

On the outer circumferential surface of the upper roll 91u, a pair of protrusions 91up, 91up is provided each predetermined angle, such as 90 degrees, in a direction of rotation Dc91 corresponding to the shape of a pair of the sealed portions 7j, 7j. Further, a cutter blade 91uc is provided at a position between the pair of protrusions 91up, 91up. On the other hand, the outer circumferential surface of the lower roll 91d has a shape capable of receiving the aforementioned pair of protrusions 91up, 91up and the cutter blade 91uc. In this example, the outer circumferential surface has a shape in which a pair of protrusions 91dp, 91dp for receiving and a receiving blade 91dc are provided at positions at which the pair of protrusions 91up, 91up and the cutter blade 91uc face, respectively. Then, these rolls 91u, 91d rotate fundamentally based on the above-described synchronization signal. That is, a servo motor is position-controlled such that the pair of protrusions 91up, 91up and the cutter blade 91uc are positioned basically at the instructed positions in the direction of rotation Dc91 indicated by the synchronization signal. Accordingly, each time when a unit of signals of the synchronization signal is output, the pair of protrusions 91up, 91up and the cutter blade 91uc rotate to face the napkin absent part 7an in the continuous sheet 7a, thereby producing the packages 7p of the napkins 1.

As in the case of the aforementioned tape-member attaching device 71, however, in the case where the state of stretch of the continuous sheet 7a changes at a positon upstream in the direction of transport from the sealing cutting device 91, it is difficult to form a pair of the sealed portions 7j, 7j accurately at the target position of the napkin absent part 7an and/or cut at a position between a pair of the sealed portions 7j, 7j, even if the sealing cutting device 91 is controlled with the synchronization signal.

Accordingly, in this example, as in the tape-member attaching device 71, the specific mark M2 formed on the continuous sheet 7a is set as the sign M2, and information on the distance of the sign M2 from the reference position, at which the sign M2 is to be originally positioned when the continuous sheet 7a is maintained with the product pitches P1, is acquired. Then, based on the information on the distance of this position, the timing in which the sealed portion 7j is formed and the timing in which the continuous sheet 7a is cut are shifted from the timing indicated by the synchronization signal. The details will be described as follows.

First, in this example, the same information as that in the tape-member attaching device 71 is used as the above-described information on the distance of the position, and further the control unit 82 (FIG. 4) performs position-control of the rotational operation of the pair of rolls 91u, 91d in consideration of the information on the distance of the position. That is, the instructed position in the direction of rotation Dc91 indicated by the synchronization signal is adjusted based on the information on the distance of the position. More specifically, the control unit 82 controls the instructed position such that, when the aforementioned information on the distance indicates that "the sign M2 is positioned downstream in the direction of transport from the reference position", the instructed position is increased by the amount of adjustment obtained by multiplying the distance by a predetermined gain, and when the information indicates that "the sign M2 is positioned upstream in the direction of transport from the reference position ", the instructed position is reduced by the amount of adjustment obtained by multiplying the distance by a predetermined gain. Accordingly, it becomes possible to accurately form a pair of the sealed portions 7j, 7j at the target position of the napkin absent part 7an, and cut at a position between the pair of sealed portions 7j, 7j.

### =First Modification =

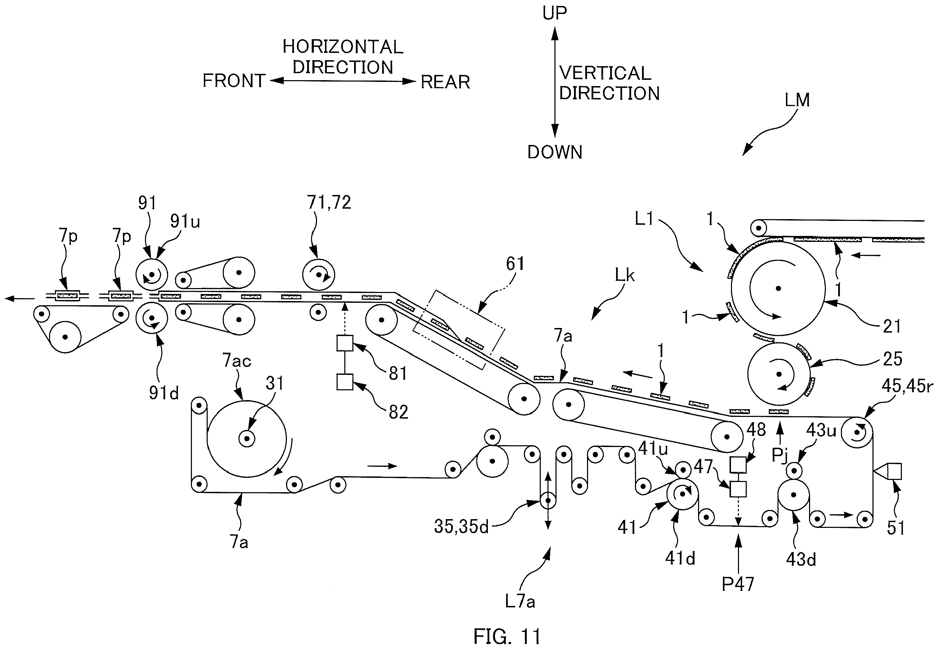

FIG. 11 is a schematic side view illustrating the manufacturing line LM of a first modification.

The main difference from the above-described embodiment (FIG. 4) is that a pair of nip rolls 43u, 43d is additionally provided at a position between the nip rolls 41u, 41d of the state-of-stretch adjusting device 41 and the roll 45r of the delivering device 45 in the packaging sheet feeding system L7a, more specifically, a position between the imaging position P47 and the roll 45r of the delivering device 45, as illustrated in FIG. 11. Except this point, the configuration is substantially the same as the above-described embodiment. Accordingly, hereinafter, this difference will be mainly described, and configurations that are similar are given the same reference numerals and descriptions thereof are omitted.

As illustrated in FIG. 11, in this first modification, due to the addition of the nip rolls 43u, 43d positioned as described above, the imaging position P47, at which the imaging device 47 captures an image, is positioned between the nip rolls 41u, 41d of the state-of-stretch adjusting device 41 and the pair of nip rolls 43u, 43d which is additionally provided at this time. Further, the pair of nip rolls 43u, 43d is supported to be rotatable about rotational axes along the CD direction, as well as is driven to rotate by a servo motor (not shown) serving as a power source. Further, the pair of nip rolls 43u, 43d is controlled so as to perform the same rotational operations as those of the aforementioned nip rolls 41u, 41d of the state-of-stretch adjusting device 41. That is, the control unit 48, which controls the pair of nip rolls 43u, 43d, controls the rotational operations of the nip rolls 43u, 43d based on the aforementioned information on the difference of the center-to-center interval, and controls the rotational operations of the nip rolls 43u, 43d also based on the above-described information on the distance of the position.

Thus, at the aforementioned imaging position P47, the state of stretch of the continuous sheet 7a is maintained substantially constant almost without changing. Accordingly, the imaging device 47 can stably image the continuous sheet 7a, resulting in enhancement in accuracy of the information on the distance of the position and the information on the difference of the center-to-center interval.

### =Second Modification=

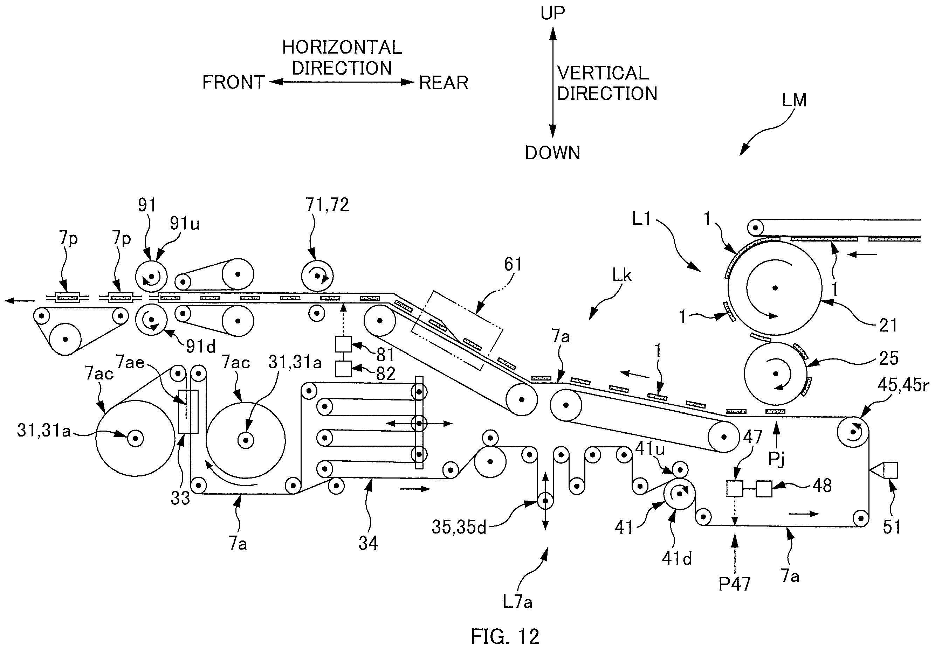

FIG. 12 is a schematic side view illustrating the manufacturing line LM of a second modification.

The main difference from the aforementioned embodiment (FIG. 4) includes two points, as illustrated in FIG. 12, (1) two feeding devices 31, 31 are provided, and (2) a material splicer 33 and an accumulating device 34 are disposed at a position between the rotational axis portion 31a of the feeding device 31 and the dancer roll 35d in the direction of transport. Except these, the configuration is substantially the same as the above-described embodiment. Accordingly, hereinafter, these differences will be mainly described, and components that are similar are given the same reference numerals and descriptions thereof are omitted.

As illustrated in FIG. 12, these two feeding devices 31, 31 include the rotational axis portions 31a, 31a, respectively. The configurations and operations of the rotational axis portions 31a are the same as those as described in the embodiment of the present disclosure. For example, the rotational axis portions 31a, 31a are driven to rotate by servo motors (not shown) serving as power sources, respectively, as well as the rotational axis portions 31a, 31a are inserted into through holes provided at the central portions in the material coils 7ac, 7ac, respectively. Then, the rotational axis portion 31a is driven to rotate so that the size of the loop is kept constant fundamentally based on the positional information of the dancer roll 35d, while the material coil 7ac is supported by the rotational axis portion 31a, thereby feeding the continuous sheet 7a of the packaging sheet from the material coil 7ac.

However, these two feeding devices 31, 31 are used in such a manner as to be switched alternately. That is, while the one feeding device 31 is feeding the continuous sheet 7a of the packaging sheet, the other feeding device 31 is substantially on standby, and when the continuous sheet 7a of the packaging sheet of the one feeding device 31 is substantially run out, the other feeding device 31 on standby starts feeding the continuous sheet 7a of the packaging sheet in place of the one feeding device 31.

The material splicer 33 is a device for connecting, to the preceding continuous sheet 7a that is being fed by the one feeding device 31, the continuous sheet 7a of the material coil 7ac that is on standby and supported by the other feeding device 31, as the succeeding continuous sheet 7a. This makes it possible to continuously feed the continuous sheet 7a of the packaging sheet without disconnection.

Such a material splicer 33 is a known component and includes, for example, a clamp mechanism (not shown) and a cutter mechanism (not shown). A connecting process is performed as follows. First, a leading end 7ae in feeding of the continuous sheet 7a of the succeeding material coil 7ac on standby in the other feeding device 31 is placed on a clamping member on one side of the clamp mechanism, as well as a double-faced tape (not shown) is attached to the leading end 7ae. Next, while the one feeding device 31 stops feeding the preceding continuous sheet 7a, the clamping member on one side is moved toward a clamping member on the other side facing this continuous sheet 7a, and these clamping members in a pair are used to clamp the continuous sheet 7a, the double-faced tape, and the leading end 7ae, thereby connecting the preceding continuous sheet 7a that stops being fed in the one feeding device 31 and the continuous sheet 7a of the succeeding material coil 7ac on standby in the other feeding device 31 (corresponding to the connecting process). Accordingly, during such a clamped state, the preceding continuous sheet 7a of the one feeding device 31 is cut, by the cutter mechanism, at a position upstream in the direction of transport from a portion connected with the double-sided tape. Thereafter, by releasing the clamped state of the pair of clamping members, the material coil 7ac that feeds the continuous sheet 7a of the packaging sheet is switched from the preceding material coil 7ac of the one feeding device 31 to the succeeding material coil 7ac of the other feeding device 31.

Here, when the succeeding continuous sheet 7a is connected to the preceding continuous sheet 7a, it is preferable to connect the succeeding continuous sheet 7a to the preceding continuous sheet 7a, while performing registration such that the distance in the direction of transport between a predetermined position of the registration mark M1 in the preceding continuous sheet 7a and a position corresponding to the aforementioned predetermined position of the registration mark M1 in the succeeding continuous sheet 7a falls within ±5% of the product pitch P1 (preferably within ±1%).

Accordingly, the continuity brought by the connection between the preceding continuous sheet 7a and the succeeding continuous sheet 7a also substantially ensures the state of registration in the registration marks M1. This makes it possible that the state-of-stretch adjusting device 41 stably adjusts the state of stretch even on the border between the preceding continuous sheet 7a and the succeeding continuous sheet 7a, and also the delivering device 45 stably adjust a transport speed value.

The accumulating device 34 is a device for accumulating the continuous sheet 7a of the packaging sheet fed from the feeding device 31, in the form of a plurality of loops such that the continuous sheet 7a can be supplied downstream in the direction of transport. Then, in the case where both the feeding devices 31, 31 stop feeding as in the case where the aforementioned material splicer 34 is used to connect the succeeding continuous sheet 7a to the preceding continuous sheet 7a, the above-described loops are made smaller so that the continuous sheet 7a can be fed to the gathering position Pj at which the continuous sheet 7a and the napkins on the napkin feeding system L1 are gathered. Accordingly, it is not necessary to stop operations of the various devices 21, 25, 71, 91 in the napkin feeding system L1 and/or the processing system Lk in association with the aforementioned connection, thereby being able to enhance productivity. Such an accumulating device 34 is known. Accordingly, more explanation is omitted.

### =Other Embodiments=

The embodiments of the present disclosure have been described hereinabove, however, the above embodiments of the present disclosure are simply to facilitate understanding of the present disclosure and are not in any way to be construed as limiting the present disclosure. The present disclosure may variously be changed or altered without departing from its gist and encompass equivalents thereof. For example, such modifications are possible as follows.

In the above-described embodiments, as illustrated in FIG. 7, the registration marks M1 are printed on the continuous sheet 7a of the packaging sheet, separately from the patterns G for design. Such registration marks M1 correspond to "mark elements" in claims. In other words, the delivering device 45 and the state-of-stretch adjusting device 41 are controlled based on the information on the amount of deviation of the position of such a registration mark M1 and the information on the amount of deviation of the center-to-center interval of the registration marks M1. However, it is not limited thereto. That is, the patterns G may be used instead of the registration marks M1.

Further, in the above-described embodiments, as illustrated in FIG. 7, the marks M2 are printed as the second mark elements on the continuous sheet 7a separately from the registration marks M1 serving as the mark elements, and the tape-member attaching device 71 and the sealing cutting device 91 are controlled using the marks M2 as the signs M2. However, it is not limited thereto. For example, instead of the marks M2, the registration marks M1 may be used also as the signs M2 to control the aforementioned devices 71, 91. That is, "mark elements" according to claim 1 may also serve as "second mark elements" according to claims 3 to 5, and further a part of the mark elements may also serve as the second mark elements.

In the above-described embodiments, the registration marks M1 serving as mark elements of claim 7 are formed by being printed on the continuous sheet 7a of the packaging sheet, however, the forming of the mark elements are not limited to printing. For example, the mark elements may be formed by pressing the continuous sheet 7a of the packaging sheet in the thickness direction to form a plurality of dotted recesses or a plurality of linear recesses.

In the above-described embodiments, the sanitary napkin 1 of FIG. 1A is described as one example of an absorbent article, but the absorbent article is not limited to the sanitary napkin 1 as long as it absorbs a wearer's excreta. For example, the absorbent article may be a disposable diaper, or a urine absorbing pad.

In the above-described embodiments, as illustrated in FIG. 4, the delivering device 45 includes the roll 45r, while the state-of-stretch adjusting device 41 includes the pair of nip rolls 41u, 41d, however they are not limited thereto. For example, the devices 41, 45 may be constituted by a belt conveyor. That is, for example, the sheet 7a may be transported by using a structure including a pair of rolls and an endless belt wound around the pair of rolls, in place of the roll 45r and the nip rolls 41u, 41d, and bringing one surface of the continuous sheet 7a into contact with the outer circumferential surface of the endless belt.

In the above-described embodiments, the imaging device 47 and the control unit 48 of FIG. 4 are used to acquire information on the amount of deviation of the position of the registration marks M1 and information on the amount of deviation of the center-to-center interval of the registration marks M1, and also the imaging device 81 and the control unit 82 of FIG. 4 are used to acquire information on the amount of deviation of the position of the sign M2. Specific example of a device capable of acquiring such information includes an image sensor CV-5000 (manufactured by KEYENCE CORPORATION). However, the device is not limited thereto. That is, a device other than the image sensor may be used, and also the aforementioned information may be acquired by using a sensor (e.g., a phototube, etc.) other than the imaging devices 47, 81.

### [Reference Signs List]

1 sanitary napkin (absorbent article), 1w wing portion,
2 top sheet, 3 absorbent body, 4 back sheet,
5a adhesive, 5b adhesive, 6a protection sheet, 6b protection sheet,
7 packaging sheet, 7Lg longer side, 7S shorter side,
7a continuous sheet of packaging sheet,
7ac material coil, 7ae leading end, 7ae1 end, 7ae2 end,
7an absent part, 7j sealed portion,
7p package,
8 tape member,
21 state-of-napkin changing device (direction changing device, pitch changing device),
22 rotating drum, 23 holding pad, 23s holding surface,
25 joining device, 26 rotating drum, 26s outer circumferential surface,
31 feeding device, 31a rotational axis portion,
33 material splicer, 34 accumulating device,
35 tension controller, 35d dancer roll,
41 state-of-stretch adjusting device, 41u nip roll, 41d nip roll,
43u nip roll, 43d nip roll,
45 delivering device, 45r roll,
47 imaging device, 48 control unit,
51 adhesive applying device,
61 folding device,
71 tape-member attaching device, 72 rotating drum (rotating body),
81 imaging device, 82 control unit,
91 sealing cutting device, 91u upper roll, 91uc cutter blade,
91up protrusion,
91d lower roll, 91dp protrusion, 91dc protrusion,
LM manufacturing line,
CV transporting apparatus,
L1 napkin feeding system, L7a packaging sheet feeding system,
Lk processing system,
Lb folding line,
G pattern, IL illustration,
M1 registration mark (mark element)
M2 sign (mark, second mark element),
Pj gathering position, P7a facing position,
Pc position,
C22 rotational axis, C23 axis, C26 rotational axis, P47 imaging position,

## Claims

1. A method for manufacturing a package formed by wrapping an absorbent article with a packaging sheet, the method comprising:
a first transport process of transporting a plurality of absorbent articles at a first pitch,
a second transport process of transporting a continuous sheet of packaging sheets in a direction of transport toward a gathering position, the gathering position being a position where the continuous sheet and the absorbent articles are gathered, the continuous sheet having mark elements wherein the mark elements are aligned in the direction of transport at a center-to-center interval that is smaller than the first pitch when the continuous sheet is in an unstretched state, and
a joining process of joining each of the absorbent articles to the continuous sheet at the gathering position,
the second transport process comprising
a state-of-stretch adjusting process of acquiring information on a difference of the center-to-center interval between the mark elements from the first pitch, and adjusting a state of stretch of the continuous sheet using a state-of-stretch adjusting device based on the information on the difference of the center-to-center interval, and
a transport speed value adjusting process of acquiring information on a distance of a position of one of the mark elements from a reference position, the reference position being based on the first pitch on the continuous sheet and adjusting, based on the information on the distance of the position, a transport speed value at a time when a delivering device delivers the continuous sheet toward the gathering position.

2. A method for manufacturing a package of an absorbent article according to claim 1, the method further comprising:
a tri-folding process of folding both lateral ends of the continuous sheet inward together with the absorbent articles using a folding device so as to form the continuous sheet in a tri-folded state, when the continuous sheet passes a position downstream in the direction of transport from the gathering position.

3. A method for manufacturing a package of an absorbent article according to claim 2, the method further comprising:
a third transport process of transporting the tri-folded continuous sheet in the direction of transport, wherein
the tri-folded continuous sheet has a plurality of second mark elements aligned in the direction of transport corresponding to the absorbent articles,
the third transport process comprises
a second position distance information acquisition process of acquiring information on a distance of a position of one of the second mark elements from a reference position, the reference position being based on the first pitch on the tri-folded continuous sheet, and
a fixing process of fixing the continuous sheet in the tri-folded state by attaching a tape member to the tri-folded continuous sheet using a tape-member attaching device, when the tri-folded continuous sheet passes a predetermined position in the direction of transport,
the tape-member attaching device is used to deliver the tape member to the predetermined position by rotating a rotating body of the tape-member attaching device while holding the tape member on an outer circumferential surface of the rotating body, and
timing in which the tape member is delivered to the predetermined position is shifted from timing indicated by a synchronization signal for processing the continuous sheet at the first pitch, based on the information on the distance.

4. A method for manufacturing a package of an absorbent article according to claim 2, the method further comprising:
a third transport process of transporting the tri-folded continuous sheet in the direction of transport, wherein
the tri-folded continuous sheet has a plurality of second mark elements aligned in the direction of transport corresponding to the absorbent articles,
the third transport process comprises
a second position distance information acquisition process of acquiring information on a distance of a position of one of the second mark elements from a reference position, the reference position being based on the first pitch on the tri-folded continuous sheet, and
a forming process of forming the package such that while a pair of sealed portions is formed in the tri-folded continuous sheet using a sealing cutting device, the continuous sheet is cut at a position between the pair of sealed portions using the sealing cutting device, and
in the forming process, timing in which the sealed portions are formed and timing in which the continuous sheet is cut by the sealing cutting device are shifted from timing indicated by a synchronization signal for processing the continuous sheet at the first pitch, based on the information on the distance.

5. A method for manufacturing a package of an absorbent article according to any one of claims 2 to 4, wherein
in the tri-folding process, the folding device is used to fold both the lateral ends inward along folding lines defined in two positions in a width direction in the continuous sheet such that the continuous sheet is formed in the tri-folded state,
the second mark elements are provided in a position between the folding lines in the continuous sheet, and
information on the distance of the position of one of the second mark elements is acquired in the second position distance information acquisition process by imaging the one of the second mark elements in the tri-folded continuous sheet using an imaging device.

6. A method for manufacturing a package of an absorbent article according to any one of claims 1 to 5, wherein
the absorbent article and the continuous sheet are joined to each other with an adhesive in the joining process,
information on the distance of the position of one of the mark elements and information on the difference of the center-to-center interval are acquired by imaging the mark elements on the continuous sheet, in a predetermined imaging position in the direction of transport using an imaging device, and
the adhesive is applied onto the continuous sheet in a position between the imaging position and the gathering position in the direction of transport using an adhesive applying device.

7. A method for manufacturing a package of an absorbent article according to any one of claims 1 to 6, the method further comprising:
a connecting process of, when the continuous sheet fed from a material coil is defined as a preceding continuous sheet, connecting a leading end in feeding of a succeeding continuous sheet fed from another material coil to the preceding continuous sheet, wherein
in the connecting process, the succeeding continuous sheet is connected to the preceding continuous sheet while performing registration such that a distance in the direction of transport between a predetermined position of one of the mark elements in the preceding continuous sheet and a position corresponding to the predetermined position of one of the mark elements in the succeeding continuous sheet falls within ±5% of the first pitch.

8. A method for manufacturing a package of an absorbent article according to any one of claims 1 to 7, wherein
in the first transport process,
a direction changing device is used to receive the absorbent articles transported from an upstream side in the direction of transport, with a longitudinal direction of the absorbent articles being along the direction of transport,
the direction changing device is used to change a direction of the received absorbent articles such that a lateral direction intersecting the longitudinal direction of the absorbent articles is directed along the direction of transport, and
the absorbent articles with the lateral direction thereof being along the direction of transport are transported toward the gathering position where the absorbent articles and the continuous sheet are gathered.

9. A method for manufacturing a package of an absorbent article according to any one of claims 1 to 8, wherein
in the first transport process,
a pitch changing device is used to receive the absorbent articles transported from the upstream side in the direction of transport at a transport pitch different from the first pitch, and
the pitch changing device changes the transport pitch of the received absorbent articles into the first pitch, to transport the absorbent articles in the direction of transport.

10. A method for manufacturing a package of an absorbent article according to any one of claims 1 to 9, wherein
in the state-of-stretch adjusting process, a state of stretch of the continuous sheet is adjusted using the state-of-stretch adjusting device also based on the information on the distance of the position.

11. An apparatus for manufacturing a package formed by wrapping an absorbent article with a packaging sheet, the apparatus comprising:
a first transporting apparatus for transporting a plurality of absorbent articles at a first pitch;
a second transporting apparatus for transporting a continuous sheet of packaging sheets in a direction of transport toward a gathering position, the gathering position being a position where the continuous sheet and the absorbent articles are gathered, the continuous sheet having mark elements wherein the mark elements are aligned in the direction of transport at a center-to-center interval that is smaller than the first pitch when the continuous sheet is in an unstretched state; and
a joining device for joining each of the absorbent articles to the continuous sheet at the gathering position,
the second transporting apparatus comprising
a state-of-stretch adjusting device for acquiring information on a difference of the center-to-center interval between the mark elements from the first pitch, and adjusting a state of stretch of the continuous sheet based on the information on the difference of the center-to-center interval; and
a delivering device for acquiring information on a distance of a position of one of the mark elements from a reference position, the reference position being based on the first pitch on the continuous sheet, and adjusting, based on the information on the distance of the position, a transport speed value at a time when delivering the continuous sheet toward the gathering position.
